(19) Europäisches Patentamt
European Patent Office
Office européen des brevets

(11) **EP 3 052 022 B1**

(12) **FASCICULE DE BREVET EUROPEEN**

(45) Date de publication et mention
de la délivrance du brevet:
**12.09.2018 Bulletin 2018/37**

(21) Numéro de dépôt: **14777068.9**

(22) Date de dépôt: **26.09.2014**

(51) Int Cl.:
*A61B 5/1455* [(2006.01)]    *G01N 33/72* [(2006.01)]
*A61B 5/00* [(2006.01)]    *A61B 5/1495* [(2006.01)]

(86) Numéro de dépôt international:
**PCT/EP2014/070605**

(87) Numéro de publication internationale:
**WO 2015/044336 (02.04.2015 Gazette 2015/13)**

(54) **DISPOSITIF ET PROCÉDÉ DE MESURE NON INVASIVE POUR L'ESTIMATION DE PARAMÈTRES MÉTABOLIQUES LOCAUX**

NICHTINVASIVE MESSVORRICHTUNG UND VERFAHREN ZUR SCHÄTZUNG VON METABOLISCHEN LOKALEN PARAMETERN

NON-INVASIVE MEASUREMENT DEVICE AND METHOD FOR ESTIMATING LOCAL METABOLIC PARAMETERS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.09.2013 FR 1359442**

(43) Date de publication de la demande:
**10.08.2016 Bulletin 2016/32**

(73) Titulaire: **APD Advanced Perfusion Diagnostics**
**69008 Lyon (FR)**

(72) Inventeurs:
• **BEUTE, Jan**
**NL-1355 HK Almere (NL)**
• **BONIDAL, Rémi**
**F-69008 Lyon (FR)**

(74) Mandataire: **Lemoine, Jean-Sébastien et al**
**Cabinet BREV&SUD**
**55, avenue Clément Ader**
**34170 Castelnau-le-Lez (FR)**

(56) Documents cités:
**US-A- 6 104 938**    **US-A1- 2001 047 128**
**US-A1- 2007 282 183**    **US-A1- 2008 200 780**
**US-A1- 2009 076 354**    **US-A1- 2009 281 403**
**US-A1- 2012 108 925**    **US-B1- 7 738 935**

**Description**

## DOMAINE TECHNIQUE

La présente invention concerne le domaine technique général des dispositifs et procédés de mesure non invasive pour l'estimation de paramètres métaboliques d'un organe d'un patient, tel que le tube digestif d'un être humain ou d'un animal.

Elle peut être utilisée dans de nombreuses applications pour protéger le patient contre des affections liées au sang, notamment thromboses, embolies, hémorragies, hémopathies et présence d'éléments anormaux dans le sang.

Elle permet notamment de fournir à l'utilisateur des informations relatives à la microcirculation splanchnique pour l'assister dans le choix de traitements adaptés au patient.

## PRESENTATION GENERALE DE L'ART ANTERIEUR

Dans le domaine médical il est souvent nécessaire de contrôler la bonne oxygénation tissulaire d'un patient.

L'oxygénation tissulaire est assurée par le sang du patient qui permet de délivrer de l'oxygène et des nutriments à toutes les parties du corps du patient. Les vaisseaux sanguins permettent la circulation du sang dans l'organisme.

La circulation sanguine peut être divisée en deux aspects : la macrocirculation et la microcirculation.

La macrocirculation comprend tous les vaisseaux d'un diamètre supérieur à 150-300 $\mu$m, tels que des artères proximales, élastiques, de gros calibre, ainsi que des artères plus distales de taille moyenne qui sont de nature musculaire. L'une des fonctions principales de la macrocirculation concerne le transport du sang du coeur vers les autres organes.

La microcirculation comprend tous les vaisseaux d'un diamètre inférieur à 150-300 $\mu$m, tels que les petites artères, les artérioles, les capillaires et les veinules. La microcirculation est une liaison entre le sang et la cellule. Par cette liaison, les tissus et les cellules sont approvisionnés en oxygène.

On connaît de nombreux dispositifs et procédés pour estimer des paramètres macrocirculatoires. Ces dispositifs et procédés permettent de guider l'utilisateur dans le choix d'un traitement permettant d'optimiser ces paramètres macrocirculatoires.

Toutefois, même si l'optimisation des paramètres macrocirculatoires est nécessaire, celle-ci n'est pas suffisante pour garantir une bonne oxygénation tissulaire du patient.

En effet, dans plusieurs circonstances rencontrées en médecine intensive, la présence d'altérations des flux microcirculatoires, à l'origine d'une hypoxie cellulaire, coexiste avec la présence de flux macrocirculatoires tout à fait adéquats.

Une meilleure connaissance des mécanismes physiopathologiques microcirculatoires associés à un monitorage plus précis de la perfusion tissulaire sont donc nécessaires pour améliorer la prise en charge des patients.

Le document US 2001/047128 A1 divulgue de combiner des données mesurées par spectrométrie à des données issues d'un oxymètre de pouls pour distinguer les concentrations en oxyhémoglobine et désoxyhémoglobine des sangs veineux et artériel. Les documents US 7 738 935 A et US 2009/076354 A1 enseignent l'utilisation d'oxymètres de pouls pour mesurer des concentrations en oxyhémoglobine et désoxyhémoglobine. Or un oxymètre de pouls permet uniquement de mesurer des paramètres relatifs au sang pulsatile, celui-ci ne permet pas de mesurer l'oxyhémoglobine et la désoxyhémoglobine dans le sang veineux.

A l'heure actuelle aucun dispositif ne permet de contrôler en temps réel et de manière peu invasive les paramètres physiopathologiques microcirculatoires d'un patient, en particulier au niveau des flux microcirculatoires.

Un but de la présente invention est de proposer un dispositif et un procédé permettant d'estimer, en temps réel, des paramètres métaboliques locaux d'un patient.

On entend, dans le cadre de la présente invention, par « *paramètres métaboliques locaux* », des paramètres métaboliques situés au voisinage de points de mesures acquis pour permettre l'estimation de ces paramètres, ce voisinage pouvant s'étendre à l'échelle d'un organe.

## PRESENTATION DE L'INVENTION

A cet effet, l'invention propose un procédé d'estimation d'au moins un paramètre métabolique local d'une zone tissulaire d'un patient, caractérisé en ce que le procédé comprend les étapes suivantes :

   ◦ Réception d'intensités lumineuses mesurées pour la zone tissulaire considérée, lesdites intensités lumineuses étant mesurées par un dispositif d'acquisition pour au moins trois longueurs d'onde différentes comprises entre 600 et 1000 nm, lesdites longueurs d'onde étant choisies en fonction de l'absorptivité de différents types d'hémoglobine, tel que l'oxyhémoglobine et la désoxyhémoglobine, l'étape de réception consistant à recevoir, pour chaque longueur d'onde, une pluralité d'intensités lumineuses mesurées à différents instants d'une période de pulsation cardiaque,

◦ Sélection, pour chaque longueur d'onde, d'intensités lumineuses mesurées à au moins un instant du cycle cardiaque pour lequel on souhaite estimer ledit et au moins un paramètre métabolique local,
◦ Calcul, à partir des intensités lumineuses sélectionnées, de valeurs d'absorbance pour les trois longueurs d'onde,
◦ Détermination, à partir des valeurs d'absorbance calculées pour les trois longueurs d'onde :

- d'une concentration en oxyhémoglobine,
- d'une concentration en désoxyhémoglobine,

en tenant compte de l'absorbance tissulaire dans l'estimation des concentrations en oxyhémoglobine et désoxyhémoglobine,
◦ Estimation d'au moins un paramètre métabolique local à partir des concentrations en oxyhémoglobine et désoxyhémoglobine

Selon l'invention l'étape de sélection consiste à sélectionner, parmi l'ensemble des intensités lumineuses mesurées, des intensités lumineuses pour lesquelles la quantité de sang artériel est minimale dans la zone tissulaire considérée, de sorte que les concentrations déterminées soient représentatives de concentration en oxyhémoglobine et désoxyhémoglobine dans le sang veineux. Le procédé selon l'invention permet ainsi d'obtenir des caractéristiques locales d'une zone tissulaire pour laquelle on souhaite estimer des paramètres métaboliques locaux. L'utilisation d'une troisième longueur d'onde additionnelle permet l'estimation des concentrations d'oxyhémoglobine et de désoxyhémoglobine en tenant compte de la perte lumineuse due aux tissus. Par ailleurs, le fait de sélectionner les intensités lumineuses mesurées à au moins un instant du cycle cardiaque permet de déterminer les concentrations en oxyhémoglobine et désoxyhémoglobine pour au moins une catégorie de sang parmi le sang veineux, le sang artériel et le sang total,

Des aspects préférés mais non limitatifs du procédé selon l'invention sont les suivants :

Bien entendu, la fréquence de mesure des valeurs d'absorbance aux trois longueurs d'ondes est très supérieure à la fréquence de pulsation cardiaque du patient, ce qui permet de mesurer une pluralité d'intensités lumineuses au cours d'une période de pulsation cardiaque,

Par ailleurs, le lecteur appréciera que l'étape de sélection peut être mise en oeuvre de manière automatique (à l'aide d'un algorithme de détection adapté) ou manuelle à partir de paramètres renseignés par le praticien, cette sélection dépendant du ou des paramètres métaboliques locaux que l'on souhaite estimer,

- l'étape de sélection consiste à sélectionner, parmi l'ensemble des intensités lumineuses mesurées, des intensités lumineuses pour lesquelles la quantité de sang artériel est maximale dans la zone tissulaire considérée, de sorte que les concentrations déterminées soient représentatives de concentration en oxyhémoglobine et désoxyhémoglobine dans le sang total ;

- l'étape d'estimation comprend l'estimation d'une consommation en oxygène dans la zone tissulaire considérée, à partir des concentrations en oxyhémoglobine et désoxyhémoglobine dans le sang total et dans le sang veineux ;

- le procédé comprend en outre une étape d'obtention, pour chacune des trois longueurs d'onde, de valeurs débruitées correspondant aux résultats d'une fonction mathématique appliquée à une pluralité de valeurs d'absorbance, l'étape de détermination consistant à calculer les concentrations en oxyhémoglobine et désoxyhémoglobine à partir des valeurs débruitées ;

Ceci permet d'améliorer la qualité d'estimation du ou des paramètres métaboliques locaux,

- l'étape de réception comprend la réception d'intensités lumineuses mesurées pour :

    ◦ une première longueur d'onde à laquelle l'absorption lumineuse de la désoxyhémoglobine est supérieure à l'absorption lumineuse de l'oxyhémoglobine, telle qu'une première longueur d'onde comprise entre 600 et 700nm, et de préférence égale à 660 nm,
    ◦ une deuxième longueur d'onde (906 nm) à laquelle l'absorption lumineuse de l'oxyhémoglobine est supérieure à l'absorption lumineuse de la désoxyhémoglobine, telle qu'une deuxième longueur d'onde comprise entre 900 et 1000nm, de préférence égale à 906 nm, et
    ◦ une troisième longueur d'onde (810 nm) correspondant à un point isobestique à laquelle les absorptions lumineuses de l'oxyhémoglobine et de la désoxyhémoglobine sont sensiblement égales, telle qu'une troisième longueur d'onde comprise entre 796 nm et 810 nm, et de préférence égale à 810 nm ;

Ceci permet de tenir compte de l'absorbance tissulaire de la zone considérée dans l'estimation du ou des paramètres métaboliques locaux,

- l'étape de réception comprend la réception d'intensités lumineuses mesurées pour une quatrième longueur d'onde comprise entre 700 nm et 800 nm, et de préférence égale à 750 nm, l'étape de calcul consistant à calculer les valeurs d'absorbance à partir des valeurs d'intensités lumineuses mesurées pour les quatre longueurs d'onde

Ceci permet d'améliorer la qualité d'estimation du ou des paramètres métaboliques locaux,

- l'étape de détermination comprend la résolution d'un système d'équations reliant un ensemble d'inconnues composé :

  ◦ de la concentration en oxyhémoglobine,
  ◦ de la concentration en désoxyhémoglobine et
  ◦ d'un coefficient d'absorption tissulaire,

  aux valeurs d'absorbances calculées auxdites et au moins trois longueurs d'onde en fonction de valeurs d'absorptivité molaires pour lesdites et au moins trois longueurs d'onde ;

- l'étape de détermination comprend la résolution d'un système suivant :

$$\begin{bmatrix} [HbO_2] \\ [Hb] \\ k \end{bmatrix} = l^{-1} \begin{bmatrix} \alpha_{v1,HbO_2} & \alpha_{v1,Hb} & \mu_{v1} \\ \alpha_{v2,HbO_2} & \alpha_{v2,Hb} & \mu_{v2} \\ \alpha_{v3,HbO_2} & \alpha_{v3,Hb} & \mu_{v3} \end{bmatrix}^{-1} \begin{bmatrix} A_{v1} \\ A_{v2} \\ A_{v3} \end{bmatrix}$$

Avec

- $\alpha_{v1,HbO_2}$, $\alpha_{v2,HbO_2}$, $\alpha_{v3,HbO_2}$ les absorptivités molaires de l'oxyhémoglobine aux trois longueurs d'onde v1, v2, v3,
- $\alpha_{v1,Hb}$, $\alpha_{v2,Hb}$, $\alpha_{v3,Hb}$ les absorptivités molaires de la désoxyhémoglobine aux trois longueurs d'onde v1, v2, v3,
- $\mu_{v1}$, $\mu_{v2}$, $\mu_{v3}$ les coefficients d'absorbtion tissulaire aux longueurs d'onde v1, v2, v3
- *l* la distance parcourue par la lumière ;

- l'étape de détermination comprend la résolution d'un système d'équations par une méthode d'optimisation régularisée,

Ceci permet d'améliorer la qualité d'estimation du ou des paramètres métaboliques locaux.

L'invention concerne également un produit programme d'ordinateur comportant un code programme enregistré sur un support de données lisible par un ordinateur pour exécuter le procédé décrit ci-dessus lorsque le programme d'ordinateur est appliqué à un ordinateur pour être exécuté.

L'invention concerne également un dispositif d'estimation d'au moins un paramètre métabolique local d'une zone tissulaire d'un patient, remarquable en ce qu'il comprend une unité de traitement programmée pour mettre en oeuvre les étapes du procédé décrit ci-dessus.

## PRESENTATION DES FIGURES

D'autres caractéristiques et avantages de l'invention ressortiront encore de la description qui suit, laquelle est purement illustrative et non limitative et doit être lue en regard des dessins annexés, sur lesquels :

- La figure 1 illustre schématiquement un dispositif d'acquisition pour mesurer des intensités lumineuses à différentes longueurs d'onde,
- La figure 2 illustre des courbes d'absorption de l'oxyhémoglobine et de la désoxyhémoglobine,
- La figure 3 illustre un exemple de courbe d'intensités lumineuses mesurées à une longueur d'onde donnée,
- La figure 4 illustre un exemple de procédé pour l'estimation de paramètres métaboliques locaux,

- La figure 5 est une représentation fonctionnelle d'éléments absorbant la lumière,
- La figure 6 illustre l'évolution en fonction du temps de concentrations en oxyhémoglobine et en désoxyhémoglobine, ainsi que d'une composante tissulaire,
- La figure 7 illustre des valeurs simulées d'absorbance pour différentes longueurs d'onde,
- La figure 8 illustre l'évolution en fonction du temps de concentrations estimées en oxyhémoglobine et désoxyhémoglobine.

## DESCRIPTION DETAILLEE

On va maintenant décrire plus en détail l'invention en référence à un dispositif et un procédé permettant d'estimer en temps réel et de manière non invasive des paramètres métaboliques du tube digestif.

Bien entendu, la présente invention ne se limite pas à l'estimation de paramètres métaboliques du tube digestif mais peut être appliquée à d'autres organes tels que les reins, le foie, ou tout autre organe sujet à une opération thérapeutique pouvant perturber sa microcirculation.

### 1. Dispositif d'acquisition

### 1.1. Structure du dispositif d'acquisition

Le dispositif d'acquisition est adapté pour :

- émettre de la lumière dans une zone tissulaire d'intérêt (i.e. pour laquelle on souhaite estimer des paramètres métaboliques locaux) à au moins trois longueurs d'onde différentes comprises entre 600 et 1000 nm, et
- capter la lumière réfléchie ayant traversée la zone tissulaire pour lesdites au moins trois longueurs d'onde.

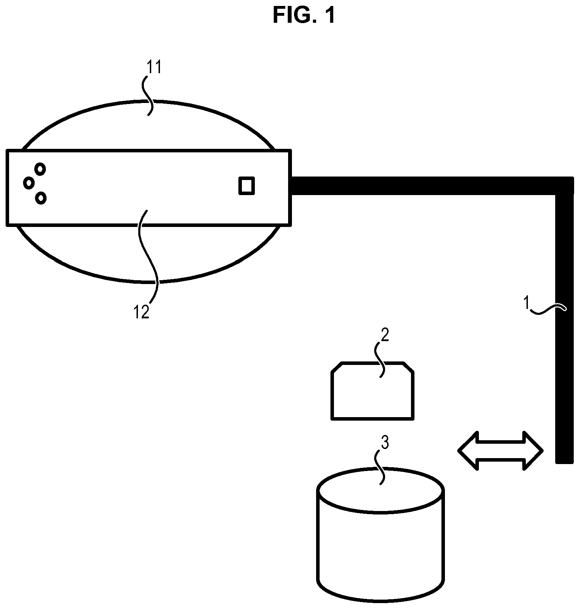

En référence à la figure 1, on a illustré un exemple de dispositif d'acquisition permettant la mesure de données nécessaires pour l'estimation de paramètres métaboliques locaux du tube digestif.

Ce dispositif est basé sur une mesure d'intensités lumineuses. Cette mesure est mise en oeuvre au niveau du tissu pour lequel on souhaite estimer des paramètres métaboliques locaux. Un tel dispositif est notamment décrit dans le document US2012/209086.

Il comprend un cathéter 1, un processeur 2 et une mémoire 3 dans laquelle un logiciel de mesure est stocké pour permettre la mise en oeuvre d'une séquence de mesures.

Le cathéter 1 comprend un des moyens de placage 11, 12 et un capteur optique à son extrémité distale.

Les moyens de placage permettent de garantir que la distance émetteur/récepteur reste inchangée et que le couple émetteur/récepteur soit immobile par rapport à la zone de mesure durant l'acquisition des signaux lumineux.

Dans le cas d'organes creux, les moyens de placage peuvent comprendre un ballon gonflable 11 et un support 12 relativement rigide sur lequel est monté le capteur optique. Le ballon gonflable 11 et le support 12 assurent un bon placage du capteur optique contre la paroi du tissu lorsque le ballon gonflable 11 est déployé. Le fait que le support 12 soit relativement rigide permet d'éviter une déformation du capteur optique, une telle déformation risquant d'induire une modification de l'agencement des éléments constituant le capteur optique.

Le capteur optique permet d'émettre une lumière dans le tissu et de recevoir de la lumière réfléchie par celui-ci. Dans le mode de réalisation illustré à la figure 1, le capteur optique comprend trois diodes électroluminescentes monochromatiques 121, 122, 123 et un récepteur 124. Le récepteur 124 permet de mesurer l'intensité lumineuse ayant traversée les différents milieux de la zone de mesure.

Chaque diode 121, 122, 123 est de préférence adaptée pour émettre un rayonnement lumineux à une longueur d'onde donnée différente de la longueur d'onde d'émission des deux autres diodes.

Avantageusement, les longueurs d'ondes d'émission des trois diodes 121, 122, 123 sont choisies en fonction de l'absorptivité des différents types d'hémoglobine - incluant notamment l'hémoglobine saturée ou « oxyhémoglobine » et l'hémoglobine non saturée ou « désoxyhémoglobine » - présents dans le tissu dont on souhaite estimer des paramètres métaboliques locaux.

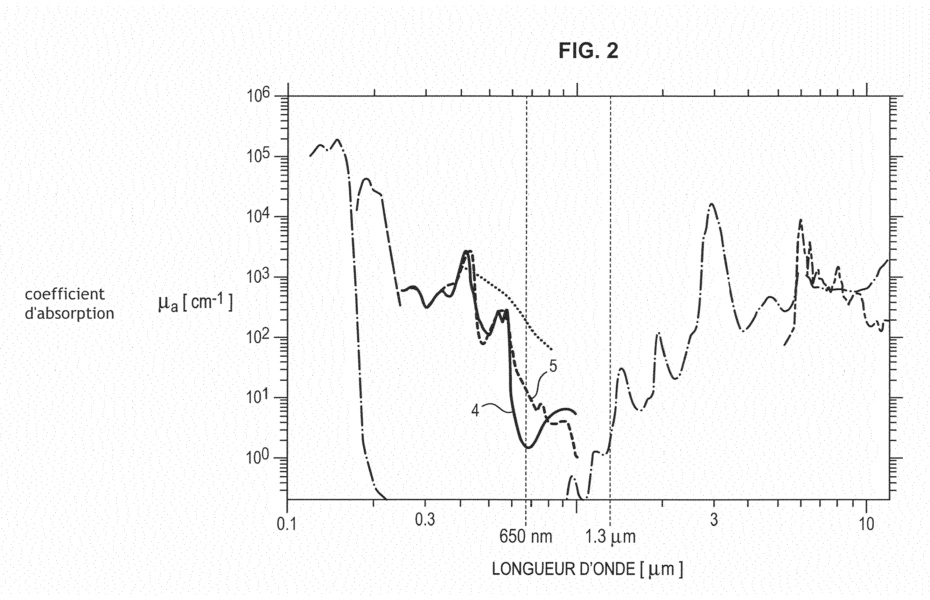

On a illustré à la figure 2 les courbes d'absorption de l'oxyhémoglobine 4 et de la désoxyhémoglobine 5. Comme on peut le constater sur ces courbes :

- l'absorption lumineuse de la désoxyhémoglobine 5 est supérieure à l'absorption lumineuse de l'oxyhémoglobine 4 dans une gamme de longueurs d'onde comprise entre 600 nm et 700 nm,
- l'absorption lumineuse de l'oxyhémoglobine 4 est supérieure à l'absorption lumineuse de la désoxyhémoglobine 5 dans une gamme de longueurs d'onde comprise entre 900 et 1000 nm.

Enfin, les absorptions lumineuses de l'oxyhémoglobine 4 et de la désoxyhémoglobine 5 sont sensiblement égales dans une gamme de longueur d'onde comprise entre 750 et 850 nm.

De préférence, les trois diodes 121, 122, 123 du capteur optique sont choisies de sorte que :

- la première diode 121 émette une lumière à une première longueur d'onde de 660 nm,
- la deuxième diode 122 émette une lumière à une deuxième longueur d'onde de 906 nm, et
- la troisième diode 123 émette une lumière à une troisième longueur d'onde correspondant à celle d'un point isobestique, c'est-à-dire comprise entre 796nm et 810 nm et de préférence égale à 810nm.

On entend, dans le cadre de la présente invention par *« point isobestique »,* une longueur d'onde ($\lambda$) à laquelle l'oxyhémoglobine et la désoxyhémoglobine possèdent sensiblement la même absorbance.

Avantageusement, le capteur optique peut comprendre une quatrième diode (non représentée) adaptée pour émettre un rayonnement lumineux à une quatrième longueur d'onde comprise entre 700 nm et 800 nm, et de préférence égale à 750 nm.

Comme il sera décrit plus en détail dans la suite, la présence d'une quatrième diode émettant à une quatrième longueur d'onde (différente des longueurs d'onde d'émission des première, deuxième et troisième diodes 121, 122, 123) permet d'améliorer la précision des calculs réalisés par le procédé décrit ci-dessous pour l'estimation de paramètres métaboliques locaux du patient. Par ailleurs, le fait de choisir une longueur d'onde comprise entre 700 et 800 nm pour la quatrième diode permet de supprimer la majorité des bruits générés par le dispositif d'acquisition.

De préférence, la distance entre chacune des diodes 121, 122, 123 du capteur optique est inférieure à cinq millimètres pour que le rayonnement émis par chacune des diodes traverse des tissus sensiblement identiques. Ceci permet de garantir une homogénéité des mesures d'intensités lumineuses réalisées aux différentes longueurs d'ondes.

*1.2. Principe de fonctionnement du dispositif d'acquisition*

Le principe de fonctionnement du dispositif décrit ci-dessus est le suivant.

Pour réaliser une mesure, le praticien déplace le cathéter 1 sur la surface d'une zone d'intérêt pour laquelle il souhaite estimer des paramètres métaboliques locaux.

Une fois le capteur optique positionné au niveau de la zone d'intérêt, le praticien commande le déploiement du ballon gonflable 11. Celui-ci se gonfle, induisant ainsi le placage du capteur optique contre la paroi de la zone d'intérêt.

Le praticien commande ensuite l'émission d'un rayonnement lumineux par le capteur optique. Chaque diode 121, 122, 123 émet alors une lumière à sa longueur d'onde d'émission. L'émission d'un rayonnement par chacune des diodes 121, 122, 123 est réalisée de manière séquentielle dans le temps, c'est-à-dire que les diodes 121, 122, 123 émettent de la lumière l'une après l'autre. Le fait que les diodes 121, 122, 123 n'émettent pas un rayonnement simultanément permet d'éviter d'éventuelles interférences au niveau du récepteur 124.

Les diodes 121, 122, 123 sont activées cycliquement à une fréquence d'activation très supérieure (i.e. entre 100 et 1000 fois supérieure) à la fréquence de pulsation cardiaque du patient. Ceci permet d'assurer la cohérence des signaux enregistrés par le récepteur 124 par rapport à la variation dans le temps des concentrations en oxyhémoglobine et en désoxyhémoglobine. Par exemple, la fréquence d'activation est de 100 Hertz.

*1.3. Mesure d'une pluralité d'intensités lumineuses*

Pour chaque longueur d'onde, on obtient ainsi une pluralité d'intensités lumineuses mesurées au court d'une période du cycle cardiaque.

Bien entendu, la mesure des intensités lumineuses (aux différentes longueurs d'ondes d'émission des diodes) peut être réalisée sur plusieurs périodes du cycle cardiaque.

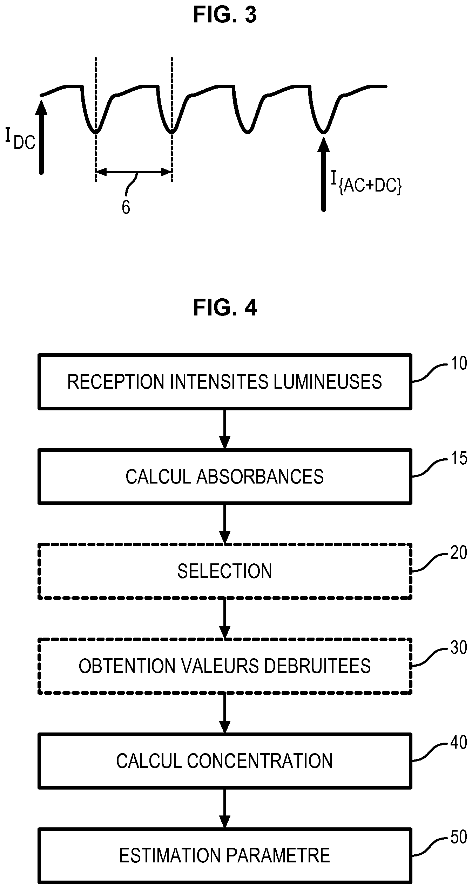

La figure 3 illustre un exemple de courbe d'intensités lumineuses mesurées à une longueur d'onde donnée pour quatre périodes 6 du cycle cardiaque.

Les intensités lumineuses mesurées à l'aide du dispositif d'acquisition décrit ci-dessus sont ensuite utilisées pour estimer les paramètres métaboliques locaux. Ces paramètres métaboliques locaux peuvent être calculés pour différents instants du cycle cardiaque en fonction des besoins du praticien.

*1.4. Utilisation des intensités lumineuses mesurées pour calculer des valeurs d'absorbance*

Le dispositif d'acquisition permet la mesure d'intensités lumineuses utilisés pour estimer des absorbances représentatives des différents milieux constituant la zone de mesure, notamment le sang veineux, le sang artériel, le sang total et les tissus. Ceci sera décrit plus en détail dans la suite.

Le sang veineux correspond à une quantité de sang non pulsé contenue en permanence dans le tissu de la zone de mesure. Le sang artériel correspond à une quantité de sang pulsé par le coeur, apportée par le flux artériel macro-circulatoire. Le sang total correspond à la présence du sang veineux et du sang artériel dans le tissu considéré.

L'absorbance correspond au résultat d'un rapport entre deux intensités lumineuses qui peuvent être mesurée(s), connu, ou estimée(s). Ces valeurs d'absorbances sont calculées pour chaque longueur d'onde.

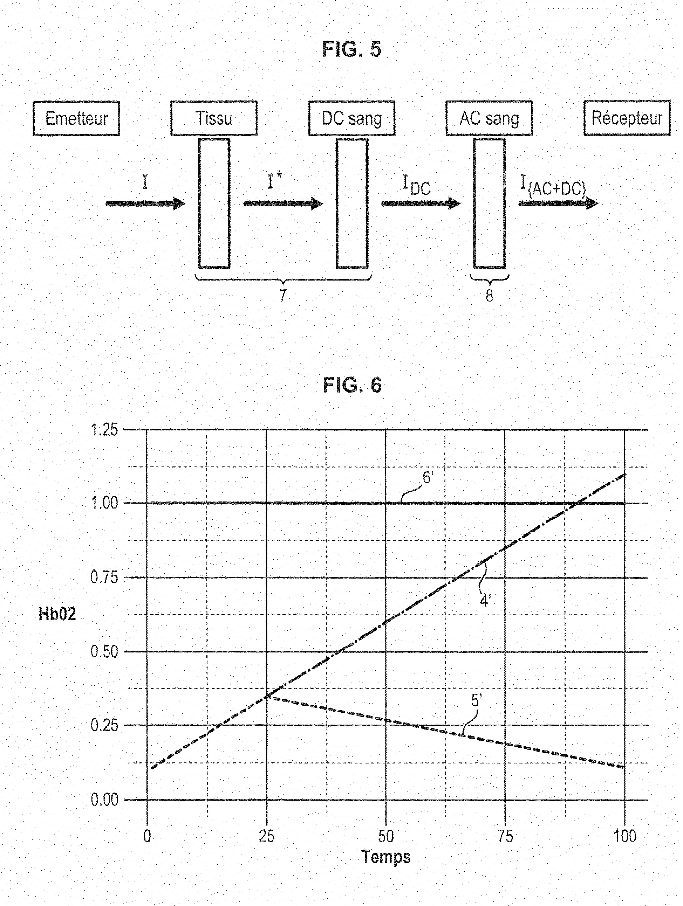

Par exemple et en référence à la figure 5, il est possible de calculer les absorbances suivante :

- l'absorbance du tissu $A_t$ qui est égale au rapport entre :

  ◦ une intensité lumineuse I\* (à une longueur d'onde donnée) correspondant à un rayonnement ayant traversé un tissu en l'absence de sang ; cette intensité lumineuse est obtenue à l'aide d'une calibration qui sera décrite dans la suite, et
  ◦ une intensité lumineuse I (à la longueur d'onde donnée) émise par l'une des diodes du capteur optique ; cette intensité lumineuse est connue,

on a alors :

$$A_t = -\log_{10}(\frac{I^*}{I})$$

- l'absorbance du sang veineux $A_{DC}$ qui est égale au rapport entre :

  ◦ l'intensité lumineuse I\*, et
  ◦ l'intensité lumineuse $I_{DC}$ correspondant à la lumière ayant traversée le tissu et le sang non pulsatile ; selon l'invention

cette intensité est estimée par sélection - parmi l'ensemble des intensités lumineuses mesurées durant une période du cycle cardiaque - de l'intensité lumineuse mesurée pour laquelle on suppose que la quantité de sang pulsé est nulle (par exemple par détection d'un maxima parmi l'ensemble des intensités lumineuses mesurées comme illustré à la figure 3),

$$A_{DC} = -\log_{10}(\frac{I_{DC}}{I^*})$$

- l'absorbance du sang artériel $A_{AC}$ qui est égale au rapport entre :

  ◦ l'intensité lumineuse $I_{DC}$, et
  ◦ l'intensité lumineuse $I_{AC+DC}$ correspondant à la lumière ayant traversée le tissu, le sang non pulsatile et le sang pulsatile ; cette intensité est estimée par sélection - parmi l'ensemble des intensités lumineuses mesurées durant une période du cycle cardiaque - de l'intensité lumineuse mesurée pour laquelle on suppose que la quantité de sang pulsé est maximale (par exemple par détection d'un minima parmi l'ensemble des intensités lumineuses mesurées comme illustré à la figure 3),

$$A_{AC} = -\log_{10}(\frac{I_{\{AC+DC\}}}{I_{DC}})$$

On peut caractériser l'absorbance totale $A_{total}$ comme la somme des trois absorbances précitées:

$$A_{total} = -\log\left(\frac{I_{\{AC+DC\}}}{I}\right) = -\log\left(\frac{I_{\{AC+DC\}}}{I_{DC}}\frac{I_{DC}}{I^*}\frac{I^*}{I}\right) = -\log\left(\frac{I_{\{AC+DC\}}}{I_{DC}}\right) - \log\left(\frac{I_{DC}}{I^*}\right) - \log\left(\frac{I^*}{I}\right)$$

$$= A_{AC} + A_{DC} + A_t$$

*2. Estimation de paramètres métaboliques locaux*

On va maintenant décrire un procédé pour l'estimation de paramètres métaboliques locaux en référence au tube digestif.

Comme illustré à la figure 4, le procédé comprend les étapes consistant à :

- recevoir 10 des intensités lumineuses mesurées par le dispositif d'acquisition pour trois longueurs d'onde différentes (ou plus),
- calculer 15 des valeurs d'absorbance à partir des intensités lumineuses mesurées,
- déterminer 40 des concentrations en oxyhémoglobine et en désoxyhémoglobine,
- estimer 50 un (ou plusieurs) paramètre(s) métabolique(s).

En référence à la figure 5, les intensités lumineuses mesurées sont fonction d'une composante statique 7 et une composante dynamique 8. La composante statique 7 est représentative de l'absorptivité lumineuse du tissu considéré ainsi que d'une quantité de sang non pulsé (i.e. sang veineux). La composante dynamique 8 représente quant à elle à l'absorptivité lumineuse du sang pulsé (i.e. sang artériel). Chacune de ces catégories de sang (sang veineux, sang artériel) comporte de l'oxyhémoglobine et de la désoxyhémoglobine dans des proportions différentes.

L'invention permet de tenir compte de l'absorptivité lumineuse du tissu par l'utilisation d'une troisième longueur d'onde correspondant au point isobestique.

Les paramètres métaboliques locaux susceptibles d'être calculés comprennent :

- les concentrations en oxyhémoglobine ($HbO_2$) et en désoxyhémoglobine (Hb), et donc un taux d'oxygénation,
- un apport en oxygène,
- une consommation en oxygène,
- un indice de perfusion, etc.

L'estimation de ces paramètres est réalisée en utilisant des valeurs d'absorbance du tube digestif obtenues à partir des intensités lumineuses mesurées pour au moins trois longueurs d'ondes par le dispositif d'acquisition décrit ci-dessus, ces longueurs d'ondes étant choisies en fonction de l'absorptivité lumineuse des différents types d'hémoglobine, et notamment l'oxyhémoglobine $HbO_2$ et la désoxyhémoglobine Hb.

*2.1. Calcul des concentrations en oxyhémoglobine HBO2 et en désoxyhémoglobine Hb*

Dans la suite, on suppose que des valeurs d'absorbance du tube digestif ont été obtenues à partir des intensités lumineuses mesurées par le dispositif d'acquisition pour les trois longueurs d'onde suivantes :

- une première longueur égale à 660nm à laquelle la désoxyhémoglobine Hb absorbe plus les rayons lumineux (que l'oxyhémoglobine),
- une deuxième longueur d'onde égale à 905 nm à laquelle l'oxyhémoglobine $HbO_2$ absorbe plus les rayons lumineux (que la désoxyhémoglobine), et
- une troisième longueur d'onde égale à 810 nm à laquelle les absorptions lumineuses de l'oxyhémoglobine $HbO_2$ et de la désoxyhémoglobine Hb sont sensiblement égales.

L'utilisation de la troisième longueur d'onde égale à 810nm permet de s'affranchir de la composante tissulaire. En effet pour chaque longueur d'onde **v**, on obtient par application de la loi de Beer-Lambert :

$$A_v = l\,(\alpha_{v,1}[1] + \alpha_{v,2}[2] + \alpha_{v,3}[3] + \mu_v k)$$

Avec :

- $A_v$: l'absorbance à la longueur d'onde v,
- $l$ la distance parcourue par la lumière,
- $\alpha_{v,1}$ l'absorptivité molaire de l'espèce 1 à la longueur d'onde v,
- $\alpha_{v,2}$ l'absorptivité molaire de l'espèce 2 à la longueur d'onde v,
- $\alpha_{v,3}$ l'absorptivité molaire de l'espèce 3 à la longueur d'onde v,
- [1] la concentration de l'espèce 1,
- [2] la concentration de l'espèce 2,
- [3] la concentration de l'espèce 3,
- $\mu_v$ le coefficient d'absorption tissulaire,
- $k$ un paramètre de calage permettant d'ajuster l'absorptivité tissulaire.

Selon la loi de Beer-Lambert, l'absorbance d'une solution est proportionnelle à la concentration des substances en solution, à condition de se placer à la longueur d'onde à laquelle la substance absorbe les rayons lumineux. C'est pourquoi la longueur d'onde est choisie en fonction de la substance dont on veut connaître la concentration. Dans le cas d'espèce, les trois longueurs d'ondes sont choisies en fonction de l'absorptivité de l'hémoglobine dans ses différents états (saturé/non saturé).

L'application de cette équation pour chacune des trois longueurs d'onde nous fournit un système de trois équations à quatre inconnues ([1], [2], [3] et k).

Or, le choix d'une longueur d'onde de 810nm (point isobestique) permet de simplifier cette relation puisqu'une des espèces (espèce 3) est composée des deux autres (espèce 1 et 2). Plus précisément, l'espèce 3 qui correspond à l'hémoglobine totale tHb est composée d'oxyhémoglobine $HbO_2$ et de désoxyhémoglobine Hb.

La concentration en hémoglobine totale [$tHb$] peut donc être définie par :

$$[HbO_2] + [Hb] = [tHb].$$

En supposant le coefficient d'absorption tissulaire $\mu_v$ constant pour toutes les longueurs d'onde considérées, on obtient le système d'équations suivant :

$$A_{660} = l \left( \alpha_{660,HbO_2} [HbO\_2] + \alpha_{660,Hb}[Hb] + \mu_{660}k \right)$$

$$A_{810} = l \left( \alpha_{810,HbO_2} [HbO_2] + \alpha_{810,Hb}[Hb] + \mu_{810}k \right)$$

$$A_{905} = l \left( \alpha_{905,HbO_2} [HbO_2] + \alpha_{905,Hb}[Hb] + \mu_{905}k \right)$$

On obtient la forme matricielle suivante :

$$\begin{bmatrix} [HbO_2] \\ [Hb] \\ k \end{bmatrix} = l^{-1} M^{-1} \begin{bmatrix} A_{660} \\ A_{810} \\ A_{905} \end{bmatrix}$$

Avec

$$M = \begin{bmatrix} \alpha_{660,HbO_2} & \alpha_{660,Hb} & \mu_{660} \\ \alpha_{810,HbO_2} & \alpha_{810,Hb} & \mu_{810} \\ \alpha_{905,HbO_2} & \alpha_{905,Hb} & \mu_{905} \end{bmatrix}$$

Toutes les grandeurs de la matrice $M$ - à savoir les différentes valeurs d'absorptivité molaire - sont des constantes qui sont obtenues expérimentalement ou par la littérature. Les valeurs d'absorbances de la matrice $\begin{bmatrix} A_{660} \\ A_{810} \\ A_{905} \end{bmatrix}$ sont quant à elles calculées à partir des intensités lumineuses mesurées à l'aide du dispositif d'acquisition décrit ci-dessus. Enfin, la distance I parcourue par la lumière est également connue, celle-ci étant fonction de la distance entre chaque diode 121, 122, 123 et le récepteur 124 (chaque distance diode/récepteur étant connue).

A titre indicatif, le tableau ci-dessous (données de W. B. Gratzer, Med. Res. Council Labs, Holly Hill, London et N. Kollias, Wellman Laboratories, Harvard Médical School, Boston) fournit les valeurs d'absorptivité molaire (en cm$^{-1}$/M) de l'oxyhémoglobine et de la désoxyhémoglobine pour différentes longueurs d'onde :

| nm | Hb02 | Hb |
|---|---|---|
| 660 | 319 | 3226 |
| 750 | 518 | 1405 |
| 810 | 864 | 718 |

(suite)

| nm | Hb02 | Hb |
|-----|------|-----|
| 850 | 1058 | 691 |
| 906 | 1209 | 770 |

La valeur du coefficient d'absorption tissulaire $\mu_v$ peut elle aussi être obtenue expérimentalement ou par la littérature et dépend du tissu considéré (tube digestif, foie, rate, coeur, etc.). Par exemple dans le cas du tube digestif, la valeur du coefficient d'absorption tissulaire $\mu_v$ peut être considérée comme égale à 1000cm$^{-1}$ pour chacune des différentes longueurs d'onde.

On peut donc calculer les concentrations en l'oxyhémoglobine [HbO$_2$] et en désoxyhémoglobine [Hb].

Avantageusement, ces concentrations peuvent être calculées pour les différentes catégories de sang (i.e. sang veineux, sang artériel ou sang total) à partir des absorbances du sang veineux A$_{DC}$, des absorbances du sang artériel A$_{DC+AC}$, ou du sang total aux différentes longueurs d'onde. Par exemple, la concentration en oxyhémoglobine dans le sang artériel peut être calculée à partir de la différence de concentration en oxyhémoglobine entre le sang total et le sang veineux. De préférence, les mesures des absorbances sont réalisées durant un temps de mesure suffisamment court pour que leur valeur soit sensiblement constante lors d'une pulsation cardiaque.

La résolution de ce système peut se faire par une résolution directe du système d'équation (3 équations/3 inconnues). Ceci permet de limiter les ressources matérielles nécessaires pour l'estimation des concentrations en l'oxyhémoglobine [HbO$_2$] et en désoxyhémoglobine [Hb].

Toutefois, un inconvénient de la résolution directe est qu'elle est sensible au bruit. Celui-ci rend la mesure instable et peut rendre le problème mal posé (i.e. sans solution ou une infinité de solutions).

Une solution pour pallier cet inconvénient consiste à résoudre ce système par une méthode d'optimisation régularisée permettant de privilégier une solution particulière dotée de propriétés qui semblent pertinentes. Une telle méthode peut être la méthode des moindres carrés régularisée en utilisant un terme de régularisation permettant de privilégier une solution particulière dotée de propriétés qui semblent pertinentes. Avantageusement ce terme de régularisation est choisi de norme 2 afin d'éviter la parcimonie dans la solution.

*2.2. Calcul sur une suite de valeurs d'absorbance*

Avantageusement, l'estimation des concentrations en oxyhémoglobine [HbO$_2$] et en désoxyhémoglobine [Hb] peut être mise en oeuvre sur des suites de valeurs d'absorbance calculées à chacune des trois longueurs d'onde. Ceci permet d'améliorer la précision de l'estimation en limitant l'influence du bruit de mesure dans le calcul des concentrations en oxyhémoglobine [HbO$_2$] et en désoxyhémoglobine [Hb].

Plus précisément, au lieu de calculer les concentrations en l'oxyhémoglobine [HbO$_2$] et en désoxyhémoglobine [Hb] à partir d'un triplet de valeurs d'absorbance ($A_{660}, A_{810} A_{905}$) pour les trois longueurs d'onde, on estime les concentrations en l'oxyhémoglobine [HbO$_2$] et en désoxyhémoglobine [Hb] à partir d'un triplet de valeurs « débruitées » ($f(Ak_{660}, n), f(Ak_{810}, n), f(Ak_{905}, n)$) pour les trois longueurs d'onde, chaque valeur « débruitée » correspondant au résultat du traitement d'une pluralité (deux, trois, quatre, dix ou plus) de valeurs d'absorbance pour la longueur d'onde considérée par une fonction linéaire ou non linéaire f

Avec :

- $Ak_v$ une « k-ième » valeur d'absorbance à la longueur d'onde v,
- $n$, le nombre de valeurs d'absorbance considéré.

La fonction linéaire ou non linéaire peut être :

- un filtre médian (fonction non linéaire),
- une moyenne glissante, ou
- tout type de fonction connue de l'homme du métier permettant, à partir d'une suite de valeurs d'absorbance, de minimiser l'impact du bruit de mesure.

Dans ce cas, le procédé comprend une étape de détermination de valeurs « débruitées » à chacune des trois longueurs d'onde (étape 30 de la figure 2).

Bien entendu, l'utilisation d'un triplet de valeurs « débruitées » suppose que les concentrations en l'oxyhémoglobine [HbO$_2$] et en désoxyhémoglobine [Hb] varient lentement par rapport à la fréquence d'échantillonnage. C'est pourquoi à une fréquence d'échantillonnage de 100 Hz, on choisira de préférence n = 5.

*2.3. Calibrage*

Dans certaines variantes de réalisation, le procédé peut comprendre une étape de calibrage.
Le calibrage permet d'évaluer la quantité de lumière absorbée par les tissus en absence de sang. Le calibrage est possible puisque la muqueuse du tube digestive est homogène par partie.
Pour effectuer ce calibrage, on positionne le dispositif d'acquisition au niveau d'une zone tissulaire d'intérêt. Ensuite, on exsangue le sang dans la zone d'intérêt, éventuellement en introduisant du sérum physiologique dans cette zone pour remplacer le sang.
En fonction des paramètres métaboliques locaux que l'on souhaite estimer, on mesure l'intensité lumineuse ayant traversée le tissu :

- pour une longueur d'onde donnée, telle qu'une longueur d'onde égale à 810 nm, ou
- pour toutes les longueurs d'onde (660nm, 810 nm, 905nm) en fonction de la nature du tissu analysé et des caractéristiques du dispositif d'acquisition.

Dans tous les cas, l'intensité mesurée $I_{exsangue}$ (à chaque longueur d'onde) est représentative de la quantité de lumière absorbée uniquement par le tissu I* puisque celui-ci ne contient plus de sang dans la zone de mesure.
Cette calibration permet ainsi de calculer, pour chaque longueur d'onde, les absorbances :

$$A_{DC} \left( A_{DC} = -\log_{10}\left(\frac{I_{DC}}{I^*}\right) \right),$$

- du sang veineux :
- du sang artériel, et
- du sang total $A_{total}$.

A partir de ces différentes catégories d'absorbance, on peut alors calculer les concentrations en oxyhémoglobine et désoxyhémoglobine :

- pour le sang veineux,
- le sang artériel, et
- le sang total

de la même manière que précédemment mais sans le 3ième paramètre de calage puisque la contribution du tissu n'est plus présente.

*2.4. Calcul d'autres paramètres métaboliques locaux*

A partir des concentrations estimées en oxyhémoglobine [HbO2] et en désoxyhémoglobine [Hb], de nombreux paramètres métaboliques locaux peuvent être calculés, tels qu'un taux d'oxygénation de la zone tissulaire.

*2.4.1. Taux d'oxygénation : première méthode de calcul*

L'estimation du taux d'oxygénation d'un sang veineux, artériel ou total $SxO_2$ peut être obtenue en appliquant la formule suivante :

$$SxO_2 = \frac{[HbO_2]_x}{[HbO_2]_x + [Hb]_x}.$$

Avec :

- $SxO_2$, le taux d'oxygénation du sang « x »,
- $[HbO_2]_x$ la concentration en oxyhémoglobine du sang « x », et
- $[Hb]_x$ la concentration en désoxyhémoglobine du sang « x », et
- où la lettre « x » désigne soit un sang veineux, artériel ou total.

Cette méthode utilise la résolution du système faisant intervenir le paramètre de calage k. A titre de remarque, la concentration en oxyhémoglobine ou en désoxyhémoglobine du sang total peut être obtenue respectivement par la somme des concentrations en oxyhémoglobine ou en désoxyhémoglobine du sang veineux et du sang artériel.

Il est à noter que le taux d'oxygénation de la zone tissulaire est équivalent au taux d'oxygénation du sang total.

### 2.4.2. Taux d'oxygénation : deuxième méthode de calcul

Le calcul de ce taux d'oxygénation peut également être obtenu par l'estimation directe de la concentration d'hémoglobine totale après avoir effectué l'étape de calibrage précédemment décrite. Ceci permet de s'affranchir du paramètre de calage k.

### 2.4.3. Calcul d'un apport en oxygène

Il est également possible de calculer l'apport en oxygène à partir de la concentration en oxyhémoglobine $[HbO_2]$ associée au sang artériel, c'est-à-dire en utilisant l'absorbance de la composante dynamique 8 de la figure 5.
L'apport en oxygène est obtenu en multipliant la concentration en oxyhémoglobine par la constante d'Hüfner.
Ici encore, l'estimation de cette concentration peut être réalisée en utilisant une concentration d'hémoglobine totale estimée à partir d'absorbance à 810 nm après avoir effectué un calibrage du dispositif d'acquisition (tel que décrit au point 2.3).

### 2.4.4. Calcul d'une consommation en oxygène

La consommation en oxygène est estimée à partir des concentrations en oxyhémoglobine du sang artériel et du sang veineux, respectivement calculées à partir des absorbances du sang artériel et du sang veineux. Pour cela, il peut être utile d'estimer l'absorbance de la composante de sang statique et d'estimer la composante de sang dynamique par l'utilisation respective de la calibration et de la sélection d'instant de mesure adéquat pendant le cycle cardiaque (voir par exemple figures 3 et 4). Pour chacune de ces absorbions, on peut appliquer une des méthodes décrites ci-dessus permettant d'obtenir les concentrations en hémoglobine saturée et en hémoglobine non saturée.
Selon une alternative, la consommation en oxygène peut être estimée à partir des concentrations en oxyhémoglobine du sang total et du sang veineux. Les concentrations en oxyhémoglobine du sang artériel peuvent être obtenues en soustrayant les concentrations en oxyhémoglobine du sang veineux au sang total.
Pour différencier la composante statique de la composante tissulaire la phase de calibration peut être utilisée.

### 2.5. Utilisation d'une quatrième longueur d'onde pour l'estimation des paramètres métaboliques locaux

Comme indiqué précédemment en référence au dispositif d'acquisition illustré à la figure 1, une mesure d'intensité lumineuse à une quatrième longueur d'onde peut être utilisée pour calculer les paramètres métaboliques locaux du patient.
L'utilisation de cette quatrième longueur d'onde permet d'améliorer la précision d'estimation des paramètres locaux calculés.
Le choix de la valeur de cette longueur d'onde supplémentaire est guidé par plusieurs facteurs :

- la longueur d'onde du point isobestique est reflet direct du volume total d'hémoglobine. En ceci, elle propose une alternative intéressante à fournir au praticien en cas de mesure bruitée pour les première, deuxième et troisième longueurs d'onde,
- il est connu que la mesure dans le domaine visible est plus sujette au bruit de mesure qu'une mesure dans l'infrarouge ; il est donc préférable de privilégier une mesure proche de l'infrarouge
- une étude du conditionnement du système montre qu'une quatrième longueur d'onde proche du rouge ou proche du point isobestique offre une meilleure robustesse au bruit de mesure lors de l'estimation des concentrations.

Le conditionnement donne une borne sur l'erreur relative commise sur la solution lorsque les paramètres du système sont modifiés. Dans le cas d'espèce, les données du problème étant des intensités lumineuses mesurées, celles-ci sont nécessairement sujette au bruit et donc entachées d'erreur.
Le tableau ci-dessous présente le conditionnement du système pour plusieurs choix de longueurs d'onde :

| nm | Conditionnement |
|---|---|
| (660,750,906) | 44 |
| (660,810,906) | 50 |
| (660,850,906) | 126 |

Au vu du tableau ci-dessus, on choisira de préférence une quatrième longueur d'onde comprise entre 700 et 800 nm, et préférentiellement égale à 750 nm. En effet, l'utilisation d'une quatrième longueur d'onde égale à 750 nm permet de minimiser le facteur de conditionnement.

*2.6. Exemple de résolution de système sur des données simulées*

On va maintenant présenter un exemple de résultats obtenus en mettant en oeuvre le procédé décrit ci-dessus. Pour cette simulation, on choisit une longueur de parcours de 1.2cm entre chaque diode 12, 122, 123 et le récepteur 124 du capteur optique.

Les coefficients d'extinctions sont ceux donnés dans le tableau d'absorptivité molaires suivant :

| nm | Hb02 | Hb |
|---|---|---|
| 660 750 810 850 906 | 319 518 864 1058 1209 | 3226 1405 718 691 770 |

La figure 6 présente l'évolution, en fonction du temps :

- des concentrations en oxyhémoglobine 4' et en désoxyhémoglobine 5', ainsi que
- de la composante tissulaire 6'.

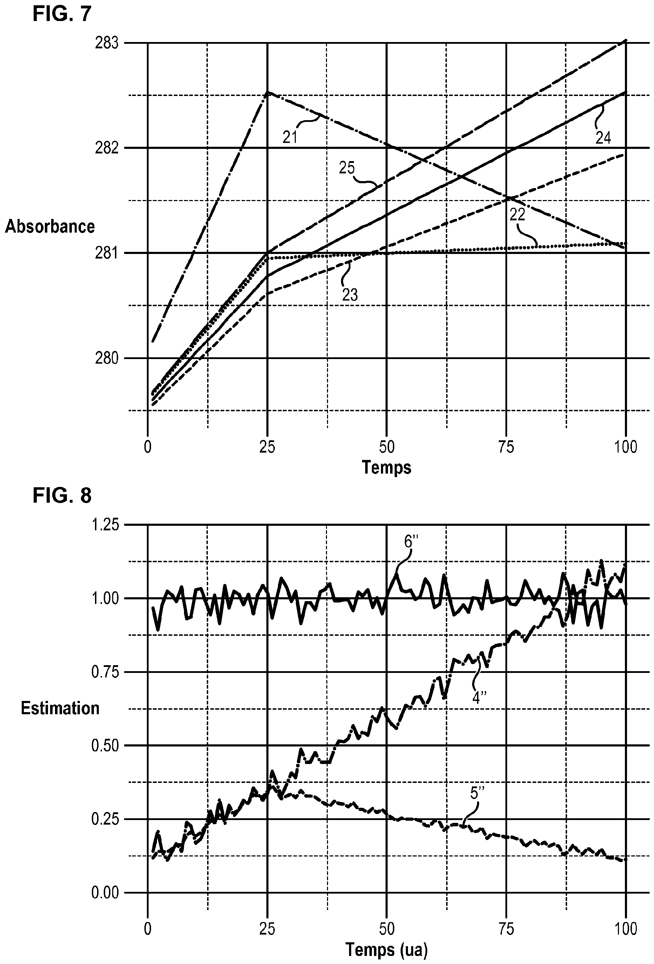

La figure 7 illustre des valeurs simulées d'absorbance pour les longueurs d'onde suivantes :

- 660 nm (référence 21),
- 750 nm (référence 22),
- 810 nm (référence 23),
- 850 nm (référence 24),
- 906 nm (référence 25).

Ces valeurs simulées d'absorbance sont obtenues par application de la loi de Beer-Lambert.

Afin d'évaluer la qualité de l'estimation des concentrations un bruit est introduit dans la mesure de l'intensité lumineuse réfléchie. Ce bruit de mesure correspond à un bruit blanc d'écart-type égal à 5% de l'intensité lumineuse reçue par le récepteur du capteur optique.

En mettant en oeuvre le procédé décrit ci-dessus, on obtient les estimations de concentrations en oxyhémoglobine 4" et désoxyhémoglobine 5" ainsi que de la composante tissulaire 6" telles qu'illustrées à la figure 8.

Une réplication sur cent individus permet d'obtenir la valeur RMS de l'erreur pour différents choix de longueurs d'onde. L'erreur RMS des simulations est présentée dans le tableau ci-dessous.

| Longueurs d'onde | HbO2 | Hb | Tissue |
|---|---|---|---|
| **(660,750,906)** | 0.030 | 0.009 | 0.039 |
| **(660,810,906)** | **0.021** | **0.007** | **0.028** |
| **(660,850,906)** | 0.062 | 0.020 | 0.086 |
| **(660,750,810,906)** | **0.020** | **0.007** | **0.024** |

Les trois premières lignes du tableau précédent sont en accord avec le tableau de conditionnement décrit plus haut et montre qu'il est préférable de considérer les triplets de fréquences (660, 750, 906) et (660, 810, 906).

L'utilisation d'une longueur d'onde additionnelle (en utilisant la méthode des moindres carrés ordinaires pour résoudre le système) permet de compléter la dernière ligne du tableau précédent. Les valeurs RMS obtenues pour cette dernière ligne confirme que l'utilisation de quatre longueurs d'onde peut apporter une redondance intéressante dans l'estimation des concentrations en oxyhémoglobine et en désoxyhémoglobine.

*3. Conclusions*

Ainsi, le procédé décrit précédemment permet d'estimer les concentrations d'oxyhémoglobine et de désoxyhémoglobine.

A partir de ces concentrations estimées, il est possible de calculer différents paramètres métaboliques tels que :

- un taux d'oxygénation à partir des concentrations en hémoglobine saturée et en hémoglobine non saturée,
- un indice de perfusion,
- un apport en oxygène,
- une consommation en oxygène, etc.

Comme indiqué précédemment, le dispositif d'acquisition active les diodes 121, 122, 123 successivement et de manière cyclique, la fréquence d'activation des diodes étant très supérieure à la fréquence de pulsation cardiaque du patient. Lorsque le dispositif d'acquisition mesure des intensités lumineuses sur une pluralité de cycles cardiaques, le procédé selon l'invention reçoit un ensemble d'intensités lumineuses mesurées sur une pluralité de cycles cardiaques pour chaque longueur d'onde.

En fonction du (ou des) paramètre(s) métabolique(s) que l'on souhaite estimer, une étape de sélection (étape 20) d'intensités lumineuses pour un (ou des) instant(s) d'intérêt d'une période du cycle cardiaque peut être mise en oeuvre. A partir de cette sélection, il est possible de calculer les concentrations en oxyhémoglobine et désoxyhémoglobine pour le sang veineux, pour le sang artériel, et pour le sang total.

La sélection d'intensités lumineuses peut être mise en oeuvre manuellement, ou de manière automatique. Dans ce cas, la sélection automatique peut être obtenue en mettant en oeuvre un algorithme de détection de minimas et/ou de maximas (et/ou d'autres points d'intérêt) dans les courbes d'intensités lumineuses mesurées aux différentes longueurs d'onde, et dont un exemple est illustré à la figure 3.

Avantageusement, le procédé décrit ci-dessus peut être implémenté sous forme de code programme pour permettre sa mise en oeuvre sur un ordinateur comprenant un processeur (configuré pour mettre en oeuvre les étapes du procédé décrit ci-dessus).

Les valeurs des paramètres métaboliques locaux calculés peuvent ensuite être affichées sur des moyens d'affichage tel qu'un écran d'ordinateur pour permettre à au praticien d'établir un diagnostic, et/ou pour le guider dans le choix d'un traitement adapté au patient.

Le lecteur aura compris que de nombreuses modifications peuvent être apportées au dispositif et procédé décrit précédemment sans sortir matériellement des nouveaux enseignements décrits ci-dessus et définis dans les revendications jointes.

Par exemple dans la description qui précède, l'absorbance du sang artériel a été définie comme fonction du rapport entre :

- l'intensité lumineuse minimale sur une période du cycle cardiaque, et
- l'intensité lumineuse maximale sur la même période du cycle cardiaque.

Le lecteur aura compris qu'une intensité lumineuse autre que l'intensité lumineuse minimale peut être sélectionnée pour calculer l'absorbance du sang artériel en tout point du cycle cardiaque.

Le lecteur aura également compris que le sang d'un patient ne comprend pas uniquement de l'oxyhémoglobine et la désoxyhémoglobine. Toutefois, dans le cadre de la présente invention, les autres éléments contenus dans ne sont pas pris en compte dans le modèle précédemment décrit, leur impact sur la qualité de l'oxygénation tissulaire d'un patient étant considérée négligeable.

## Revendications

1. Procédé d'estimation d'au moins un paramètre métabolique local d'une zone tissulaire d'un patient, comprenant les étapes suivantes :

   ◦ Réception (10) d'intensités lumineuses mesurées pour la zone tissulaire considérée, lesdites intensités lumineuses étant mesurées par un dispositif d'acquisition pour au moins trois longueurs d'onde différentes comprises entre 600 et 1000 nm, lesdites longueurs d'onde étant choisies en fonction de l'absorptivité de différents types d'hémoglobine, tel que l'oxyhémoglobine et la désoxyhémoglobine, l'étape de réception consistant à recevoir, pour chaque longueur d'onde, une pluralité d'intensités lumineuses mesurées à différents instants d'une période (8) de pulsation cardiaque,
   ◦ Sélection (20), pour chaque longueur d'onde, d'intensités lumineuses mesurées à au moins un instant du cycle cardiaque pour lequel on souhaite estimer ledit et au moins un paramètre métabolique local,
   ◦ Calcul (15), à partir des intensités lumineuses sélectionnées, de valeurs d'absorbance pour les trois longueurs d'onde,
   ◦ Détermination (40), à partir des valeurs d'absorbance calculées pour les trois longueurs d'onde :

      - d'une concentration en oxyhémoglobine,

- d'une concentration en désoxyhémoglobine,

en tenant compte de l'absorbance tissulaire dans l'estimation des concentrations en oxyhémoglobine et désoxyhémoglobine,

∘ Estimation (50) d'au moins un paramètre métabolique local à partir des concentrations en oxyhémoglobine et désoxyhémoglobine calculées,

**caractérisé en ce que**

l'étape de sélection consiste à sélectionner, parmi l'ensemble des intensités lumineuses mesurées, des intensités lumineuses pour lesquelles la quantité de sang artériel est minimale dans la zone tissulaire considérée, de sorte que les concentrations déterminées soient représentatives de concentration en oxyhémoglobine et désoxyhémoglobine dans le sang veineux

2. Procédé selon la revendication 1, dans lequel l'étape de sélection consiste de plus à sélectionner, parmi l'ensemble des intensités lumineuses mesurées, des intensités lumineuses pour lesquelles la quantité de sang artériel est maximale dans la zone tissulaire considérée, de sorte que les concentrations déterminées soient représentatives de concentration en oxyhémoglobine et désoxyhémoglobine dans le sang total.

3. Procédé selon la revendication 3, dans lequel l'étape de l'étape d'estimation comprend l'estimation d'une consommation en oxygène dans la zone tissulaire considérée, à partir des concentrations en oxyhémoglobine et désoxyhémoglobine dans le sang total et dans le sang veineux.

4. Procédé selon l'une quelconque des revendications précédentes, lequel comprend en outre une étape d'obtention (30), pour chacune des trois longueurs d'onde, de valeurs débruitées correspondant aux résultats d'une fonction mathématique appliquée à une pluralité de valeurs d'absorbance, l'étape de détermination consistant à calculer les concentrations en oxyhémoglobine et désoxyhémoglobine à partir des valeurs débruitées.

5. Procédé selon l'une quelconque des revendications précédentes, dans lequel l'étape de réception comprend la réception d'intensités lumineuses mesurées pour :

- une première longueur d'onde à laquelle l'absorption lumineuse de la désoxyhémoglobine est supérieure à l'absorption lumineuse de l'oxyhémoglobine, telle qu'une première longueur d'onde comprise entre 600 et 700nm, et de préférence égale à 660 nm,
- une deuxième longueur d'onde (906 nm) à laquelle l'absorption lumineuse de l'oxyhémoglobine est supérieure à l'absorption lumineuse de la désoxyhémoglobine, telle qu'une deuxième longueur d'onde comprise entre 900 et 1000nm, de préférence égale à 906 nm, et
- une troisième longueur d'onde (810 nm) correspondant à un point isobestique à laquelle les absorptions lumineuses de l'oxyhémoglobine et de la désoxyhémoglobine sont sensiblement égales, telle qu'une troisième longueur d'onde comprise entre 796 nm et 810 nm, et de préférence égale à 810 nm.

6. Procédé selon l'une quelconque des revendications précédentes, dans lequel l'étape de réception comprend la réception d'intensités lumineuses mesurées pour une quatrième longueur d'onde comprise entre 700 nm et 800 nm, et de préférence égale à 750 nm, l'étape de calcul consistant à calculer les valeurs d'absorbance à partir des valeurs d'intensités lumineuses mesurées pour les quatre longueurs d'onde.

7. Procédé selon l'une quelconque des revendications précédentes, dans lequel l'étape de détermination comprend la résolution d'un système d'équations reliant un ensemble d'inconnues composé :

- de la concentration en oxyhémoglobine,
- de la concentration en désoxyhémoglobine et
- d'un coefficient d'absorption tissulaire,

aux valeurs d'absorbances calculées auxdites et au moins trois longueurs d'onde en fonction de valeurs d'absorptivité molaires pour lesdites et au moins trois longueurs d'onde.

8. Procédé selon la revendication précédente, dans lequel l'étape de détermination comprend la résolution d'un système suivant :

$$\begin{bmatrix} [HbO_2] \\ [Hb] \\ k \end{bmatrix} = l^{-1} \begin{bmatrix} \alpha_{v1,HbO_2} & \alpha_{v1,Hb} & \mu_{v1} \\ \alpha_{v2,HbO_2} & \alpha_{v2,Hb} & \mu_{v2} \\ \alpha_{v3,HbO_2} & \alpha_{v3,Hb} & \mu_{v3} \end{bmatrix}^{-1} \begin{bmatrix} A_{v1} \\ A_{v2} \\ A_{v3} \end{bmatrix}_{-1}$$

Avec

- $\alpha_{v1,HbO2}$, $\alpha_{v2,HbO2}$, $\alpha_{v3,HbO2}$ les absorptivités molaires de l'oxyhémoglobine aux trois longueurs d'onde v1, v2, v3,
- $\alpha_{v1,Hb}$, $\alpha_{v2,Hb}$, $\alpha_{v3,Hb}$ les absorptivités molaires de la désoxyhémoglobine aux trois longueurs d'onde v1, v2, v3,
- $\mu_{v1}$, $\mu_{v2}$, $\mu_{v3}$ les absorptivités molaires de la zone tissulaire aux longueurs d'onde v1 , v2, v3
- l la distance parcourue par la lumière.

9. Procédé selon l'une quelconque des revendications précédentes, dans lequel l'étape de détermination comprend la résolution d'un système d'équations par une méthode d'optimisation régularisée.

10. Produit programme d'ordinateur comportant un code programme enregistré sur un support de données lisible par un ordinateur pour exécuter le procédé selon l'une quelconque des revendications précédentes lorsque le programme d'ordinateur est appliqué à un ordinateur pour être exécuté.

11. Dispositif d'estimation d'au moins un paramètre métabolique local d'une zone tissulaire d'un patient, **caractérisé en ce qu'**il comprend une unité de traitement programmée pour mettre en oeuvre les étapes du procédé selon l'une quelconque des revendications 1 à 9.

**Patentansprüche**

1. Verfahren zum Schätzen von mindestens einem lokalen Stoffwechselparameter einer Gewebezone eines Patienten, umfassend folgende Schritte:

◦ Empfangen (10) der gemessenen Lichtintensitäten für die betroffene Gewebezone, wobei die Lichtintensitäten mittels einer Erfassungsvorrichtung für mindestens drei verschiedenen Wellenlängen zwischen 600 und 1000 nm gemessen werden, wobei die Wellenlängen in Abhängigkeit des Absorptionsvermögens von verschiedenen Arten Hämoglobin, wie Oxyhämoglobin und Desoxyhämoglobin, ausgewählt werden, wobei der Schritt des Empfangens darin besteht, für jede Wellenlänge eine Vielzahl von zu unterschiedlichen Zeitpunkten einer Periode (8) des Herzschlags gemessenen Lichtintensitäten zu empfangen,
◦ Auswählen (20) für jede Wellenlänge der zu mindestens einem Zeitpunkt im Herzzyklus gemessenen Lichtintensitäten, für den es erwünscht ist, den besagten und zumindest einen lokalen Stoffwechselparameter zu schätzen,
◦ Berechnen (15) von Absorptionswerten für die drei Wellenlängen aus den ausgewählten Lichtintensitäten,
◦ Bestimmen (40) aus den für die drei Wellenlängen berechneten Absorptionswerten :

- einer Konzentration von Oxyhämoglobin,
- einer Konzentration von Desoxyhämoglobin,

unter Berücksichtigung der Gewebeabsorption bei der Schätzung der Konzentrationen von Oxyhämoglobin und Desoxyhämoglobin,
◦ Schätzen (50) von mindestens einem lokalen Stoffwechselparameter aus den berechneten Konzentrationen von Oxyhämoglobin und Desoxyhämoglobin, **dadurch gekennzeichnet, dass** der Schritt des Auswählens darin besteht, aus dem Satz von gemessenen Lichtintensitäten diejenige Lichtintensitäten auszuwählen, für die die arterielle Blutmenge in der betroffene Gewebezone minimal ist, so dass die ermittelten Konzentrationen repräsentativ für die Konzentration von Oxyhämoglobin und Desoxyhämoglobin in dem venösen Blut sind,

2. Verfahren nach Anspruch 1, bei dem der Schritt des Auswählens außerdem darin besteht, aus dem Satz von gemessenen Lichtintensitäten diejenige Lichtintensitäten auszuwählen, für die die arterielle Blutmenge in der betroffene Gewebezone maximal ist, so dass die ermittelten Konzentrationen repräsentativ für die Konzentration von Oxyhämoglobin und Desoxyhämoglobin im Vollblut sind.

3. Verfahren nach Anspruch 3, bei dem der Schritt des Schätzens das Schätzen eines Sauerstoffverbrauchs in der betroffenen Gewebezone aus den Konzentrationen von Oxyhämoglobin und Deoxyhämoglobin im Vollblut und im venösen Blut umfasst.

4. Verfahren nach iregendeinem der vorhergehenden Ansprüche, das außerdem einen Schritt des Erhaltens (30) für jede der drei Wellenlängen von rauschunterdrückten Werten umfasst, die den Ergebnissen einer auf eine Mehrzahl von Absorptionswerten angewendeten mathematischen Funktion entsprechen, wobei der Schritt des Bestimmens darin besteht, die Konzentrationen von Oxyhämoglobin und Deoxyhämoglobin aus den rauschunterdrückten Werten zu berechnen.

5. Verfahren nach irgendeinem der vorhergehenden Ansprüche, bei dem der Schritt des Empfangens das Empfangen von Lichtintensitäten umfasst, die gemessen werden für:

- eine erste Wellenlänge, bei der die Lichtabsorption von Desoxyhämoglobin größer ist als die Lichtabsorption von Oxyhämoglobin, wie eine erste Wellenlänge zwischen 600 und 700 nm und vorzugsweise gleich 660 nm,
- eine zweite Wellenlänge (906 nm), bei der die Lichtabsorption des Oxyhämoglobins größer ist als die Lichtabsorption des Desoxyhämoglobins, wie eine zweite Wellenlänge zwischen 900 und 1000 nm, vorzugsweise gleich 906 nm, und
- eine einem isobestischen Punkt entsprechende dritte Wellenlänge (810 nm), bei der die Lichtabsorptionen von Oxyhämoglobin und Desoxyhämoglobin im Wesentlichen gleich sind, wie eine dritte Wellenlänge zwischen 796 nm und 810 nm, und vorzugsweise gleich 810 nm.

6. Verfahren nach irgendeinem der vorhergehenden Ansprüche, bei dem der Schritt des Empfangens das Empfangen von für eine vierte Wellenlänge zwischen 700 nm und 800 nm und vorzugsweise gleich 750 nm gemessenen Lichtintensitäten umfasst, wobei der Schritt des Berechnens darin besteht, die Absorptionswerte aus den für die vier Wellenlängen gemessenen Lichtintensitätswerten zu berechnen.

7. Verfahren nach irgendeinem der vorhergehenden Ansprüche, bei dem der Schritt des Bestimmens das Lösen eines Systems von Gleichungen umfasst, das einen Satz von Unbekannten, der zusammengesetzt ist aus:

- der Konzentration von Oxyhämoglobin,
- der Konzentration von Deoxyhämoglobin, und
- einem Gewebeabsorptionskoeffizienten,

verbindet mit den bei den genannten und mindestens drei Wellenlängen in Abhängigkeit von den molaren Absorptionswerten für die besagten und mindestens drei Wellenlängen berechneten Absorptionswerten.

8. Verfahren nach dem vorhergehenden Anspruch, bei dem der Schritt des Bestimmens die Auflösung eines folgenden Systems umfasst:

$$\begin{bmatrix} [HbO_2] \\ [Hb] \\ k \end{bmatrix} = l^{-1} \begin{bmatrix} \alpha_{v1,HbO_2} & \alpha_{v1,Hb} & \mu_{v1} \\ \alpha_{v2,HbO_2} & \alpha_{v2,Hb} & \mu_{v2} \\ \alpha_{v3,HbO_2} & \alpha_{v3,Hb} & \mu_{v3} \end{bmatrix}^{-1} \begin{bmatrix} A_{v1} \\ A_{v2} \\ A_{v3} \end{bmatrix}_{-1}$$

wo

• $\alpha v1, HbO2, \alpha v2, HbO2, \alpha v3, HbO2$ die molaren Absorptionskoeffizienten von Oxyhämoglobin bei den drei Wellenlängen v1, v2, v3 sind,
• $\alpha v1, Hb, \alpha v2, Hb, \alpha v3, Hb$ die molaren Absorptionskoeffizienten von Desoxyhämoglobin bei den drei Wellenlängen v1, v2, v3 sind,
• $\mu v1, \mu v2, \mu v3$ die molaren Absorptionsvermögen der Gewebezone bei den Wellenlängen v1, v2, v3 sind,
• l die vom Licht zurücklegte Entfernung ist.

9. Verfahren nach irgendeinem der vorhergehenden Ansprüche, bei dem der Schritt des Bestimmens das Lösen eines Gleichungssystems mittels eines regulierten Optimierungsverfahrens umfasst.

10. Computerprogrammprodukt, umfassend einen auf einem computerlesbaren Datenträger gespeicherten Programmcode zur Durchführung des Verfahrens nach irgendeinem der vorhergehenden Ansprüche wenn das Computerprogramm auf einem Computer angewendet wird, um ausgeführt zu werden.

11. Vorrichtung zum Schätzen von mindestens einem lokalen Stoffwechselparameter einer Gewebezone eines Patienten, **dadurch gekennzeichnet, dass** sie eine Verarbeitungseinheit umfasst, die dazu programmiert ist, die Schritte des Verfahrens nach irgendeinem der Ansprüche 1 bis 9 durchzuführen.

**Claims**

1. A method for estimating at least one local metabolic parameter of a tissue area of a patient, comprising the following steps:

   ◦ receiving (10) light intensities measured for the tissue area being considered, said light intensities being measured by an acquisition device for at least three different wavelengths between 600 and 1000 nm, said wavelengths being chosen depending on the absorptivity of different types of hemoglobin, such as oxyhemoglobin and deoxyhemoglobin, the step of receiving consisting in receiving, for each wavelength, a plurality of light intensities measured at different times of a period (8) of heart beat,
   ◦ selecting (20), for each wavelength, light intensities measured at least at one time of the cardiac cycle for which it is desired to estimate said and at least one local metabolic parameter,
   ◦ calculating (15), from the selected light intensities, absorbance values for the three wavelengths,
   ◦ determining (40), from the absorbance values calculated for the three wavelengths:

      - a concentration of oxyhemoglobin,
      - a concentration of deoxyhemoglobin,

   taking into consideration the tissue absorbance in the estimation of concentrations of oxyhemoglobin and deoxyhemoglobin,
   ◦ estimating (50) at least one local metabolic parameter from the calculated concentrations of oxyhemoglobin and deoxyhemoglobin, wherein the step of selecting consists in selecting, from the set of measured light intensities, light intensities for which the amount of arterial blood is minimal in the tissue area being considered, so that the determined concentrations are representative of the concentration of oxyhemoglobin and deoxyhemoglobin in the venous blood.

2. The method according to claim 1, wherein the step of selecting consists in addition in selecting, among the set of measured light intensities, light intensities for which the amount of arterial blood is maximum in the tissue area being considered, so that the determined concentrations are representative of the concentration of oxyhemoglobin and deoxyhemoglobin in the total blood.

3. The method according to claim 3, wherein the step of estimating comprises estimating an oxygen consumption in the tissue area being considered, from the concentrations of oxyhemoglobin and deoxyhemoglobin in the total blood and in the venous blood.

4. The method according to any one of the preceding claims, which further comprises a step of obtaining (30), for each of the three wavelengths, noise-suppressed values corresponding to the results of a mathematical function applied to a plurality of absorbance values, the step of determining consisting in calculating the concentrations of oxyhemoglobin and deoxyhemoglobin from the noise-suppressed values.

5. The method according to any one of the preceding claims, wherein the step of receiving comprises receiving light intensities measured for:

   - a first wavelength at which the light absorption of deoxyhemoglobin is higher than the light absorption of oxyhemoglobin, such as a first wavelength between 600 and 700 nm, and preferably equal to 660 nm,
   - a second wavelength (906 nm) at which the light absorption of oxyhemoglobin is higher than the light absorption of deoxyhemoglobin, such as a second wavelength between 900 and 1000 nm, preferably equal to at 906 nm, and
   - a third wavelength (810 nm) corresponding to an isobestic point at which the light absorptions of oxyhemoglobin and deoxyhemoglobin are substantially equal, such as a third wavelength between 796 nm and 810 nm, and preferably 810 nm.

6. The method according to any one of the preceding claims, wherein the step of receiving comprises receiving light intensities measured for a fourth wavelength between 700 nm and 800 nm, and preferably equal to 750 nm, the step of calculating consisting in calculating the absorbance values from the light intensity values measured for the four wavelengths.

7. The method according to any one of the preceding claims, wherein the step of determining comprises solving a system of equations connecting a set of unknowns comprised of:

    - the concentration of oxyhemoglobin,
    - the concentration of deoxyhemoglobin and
    - a tissue absorption coefficient,

with the absorbance values calculated at said and at least three wavelengths depending on the molar absorptivity values for said and at least three wavelengths.

8. The method according to the preceding claim, wherein the step of determining comprises the resolution of a following system:

$$
\begin{bmatrix} [HbO_2] \\ [Hb] \\ k \end{bmatrix} = l^{-1} \begin{bmatrix} \alpha_{v1,HbO_2} & \alpha_{v1,Hb} & \mu_{v1} \\ \alpha_{v2,HbO_2} & \alpha_{v2,Hb} & \mu_{v2} \\ \alpha_{v3,HbO_2} & \alpha_{v3,Hb} & \mu_{v3} \end{bmatrix}^{-1} \begin{bmatrix} A_{v1} \\ A_{v2} \\ A_{v3} \end{bmatrix}_{-1}
$$

with

- $\alpha_{v1,HbO2}$, $\alpha_{v2,HbO2}$, $\alpha_{v3,HbO2}$ the molar absorptivities of oxyhemoglobin at the three wavelengths v1, v2, v3,
- $\alpha_{v1,Hb}$, $\alpha_{v2,Hb}$, $\alpha_{v3,Hb}$ the molar absorptivities of deoxyhemoglobin at the three wavelengths v1, v2, v3,
- $\mu_{v1}$, $\mu_{v2}$, $\mu_{v3}$ the molar absorptivities of the tissue area at the wavelengths v1, v2, v3
- l the distance traveled by the light.

9. The method according to any of the preceding claims, wherein the step of determining comprises solving a system of a system of equations by a regularized optimization method.

10. A computer program product having a program code recorded on a computer readable data medium for performing the method according to any one of the preceding claims when the computer program is applied to a computer to be executed.

11. A device for estimating at least one local metabolic parameter of a tissue area of a patient, wherein it comprises a processing unit programmed to implement the steps of the method according to any one of the claims 1 to 9.

# FIG. 1

FIG. 2

coefficient
d'absorption

$\mu_a$ [ cm⁻¹ ]

650 nm    1.3 μm

LONGUEUR D'ONDE [ μm ]

# FIG. 3

# FIG. 4

## FIG. 5

| Emetteur | | Tissu | | DC sang | | AC sang | | Récepteur |
|----------|--|-------|--|---------|--|---------|--|-----------|

$I \longrightarrow$      $I^* \longrightarrow$      $I_{DC} \longrightarrow$      $I_{\{AC+DC\}} \longrightarrow$

7          8

## FIG. 6

HbO2

6'
4'
5'

Temps

**FIG. 7**

**FIG. 8**

## EP 3 052 022 B1

**Documents brevets cités dans la description**

- US 2001047128 A1
- US 7738935 A
- US 2009076354 A1
- US 2012209086 A

**Littérature non-brevet citée dans la description**

- **DE W. B. GRATZER.** Med. Res. Council Labs. Wellman Laboratories, Harvard Médical School